# EUROPEAN PATENT SPECIFICATION

(11) **EP 0 421 760 B1**
(45) Date of publication and mention of the grant of the patent: **23.03.1994**
(21) Application number: 90310824.9
(22) Date of filing: 03.10.1990
(51) Int. Cl.: A61L 2/26, C12Q 1/22

(54) **Sterilizer test pack**
Sterilisator-Testpackung
Dispositif de test pour stérilisateur

(30) Priority: 03.10.1989 US 416699
(43) Date of publication of application: 10.04.1991
(73) Proprietor: JOHNSON & JOHNSON MEDICAL, INC., New Brunswick, New Jersey 08933-7033 (US)
(72) Inventor: Lin, Szu-Min, Arlington, Texas 76018 (US)
(74) Representative: Mercer, Christopher Paul

(56) References cited:
- EP-A- 0 069 037

## Description

### Background of the Invention

### Field of the Invention

This invention relates to the field of sterilization processes and in particular to indicators for sterilization cycles.

### Description of the Prior Art

In the field of sterilization of articles, it is desirable to determine whether a particular load of articles that is subjected to a sterilizing environment (i.e. gasses such as hydrogen peroxide or ethylene oxide or other environments such as a plasma) has, in fact, been exposed to an environment which would have killed microorganisms. For example, U.S. Patent No. 4,643,876 issued February 17, 1987, the disclosure of which is incorporated herein by reference, discloses a method of sterilizing using a plasma which uses hydrogen peroxide as the chemical precursor. It is desirable to have a simple, quick test to determine whether the sterilization environment reached the more secluded areas of the objects placed within the sterilizer.

One way of testing is to use a biological indicator as shown, for example, by U.S. Patent No. 4,461,837 to Karle et al. The indicator comprises a container having test spores and a growth medium therein. The container includes a tortuous path from the exterior to the interior of the container. The indicator is inserted into the test chamber with the load and exposed to the sterilizing environment for the sterilizing cycle. The tortuous path is intended to resist the passage of the sterilizing medium to simulate a difficult to reach portion of the load. After exposure, the growth medium and spores are mixed and incubated to determine if any spores survived.

U.S. Patent No. 4,636,472, discloses a disposable biological test pack. The pack has a porous base pad and porous pads disposed on opposite sides of a biological indicator. European Patent Application 0254,428 discloses an ethylene oxide test pack. The pack has a clear plastic tray containing a sterilization sensitive ink which has been imprinted on a card. The tray also contains a syringe having a biological indicator contained therein. Absorbent paper is placed on top of the syringe. Eurpoean Patent Application 0,255,229 shows a similar device for use with steam sterilization systems.

### Summary of the Invention

The invention relates to a test pack having a housing defining an inner volume and an opening for communication between the inner volume and the environment outside of the housing. A divider divides the inner volume into two portions. A first portion of the inner volume has an absorber positioned therein to retard passage of the sterilization atmosphere or a chemical precursor of the sterilization atmosphere (hereinafter referred to jointly as "sterilization atmosphere" or "atmosphere"). A chemical indicator to indicate exposure to the atmosphere may also be positioned within the first portion. The second portion defines a blind reservoir which contains a biological indicator for testing for successful completion of the sterilization procedure.

### Brief Description of the Drawings

The preferred embodiment will now be described with references to the accompanying drawings, wherein:
FIGURE 1 is a front-top perspective view of the test pack of the invention;
FIGURE 2 is an exploded view of the test pack of FIG. 1;
FIGURE 3 is a top plan view of the test pack; and
FIGURE 4 is a cross-sectional view along lines 4-4 of FIG. 3.

### Description of the Preferred Embodiment

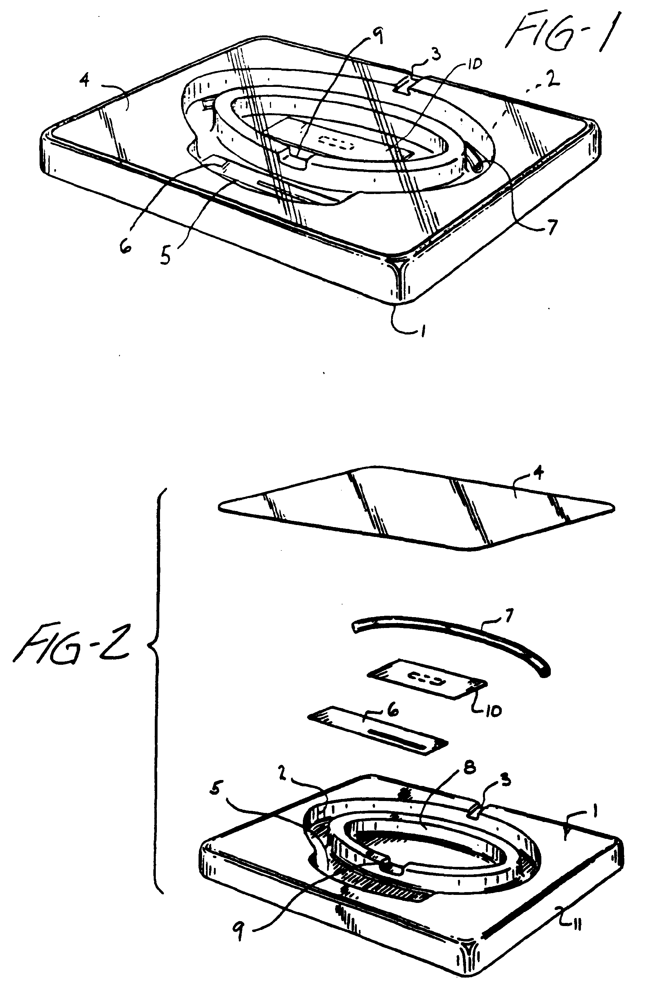

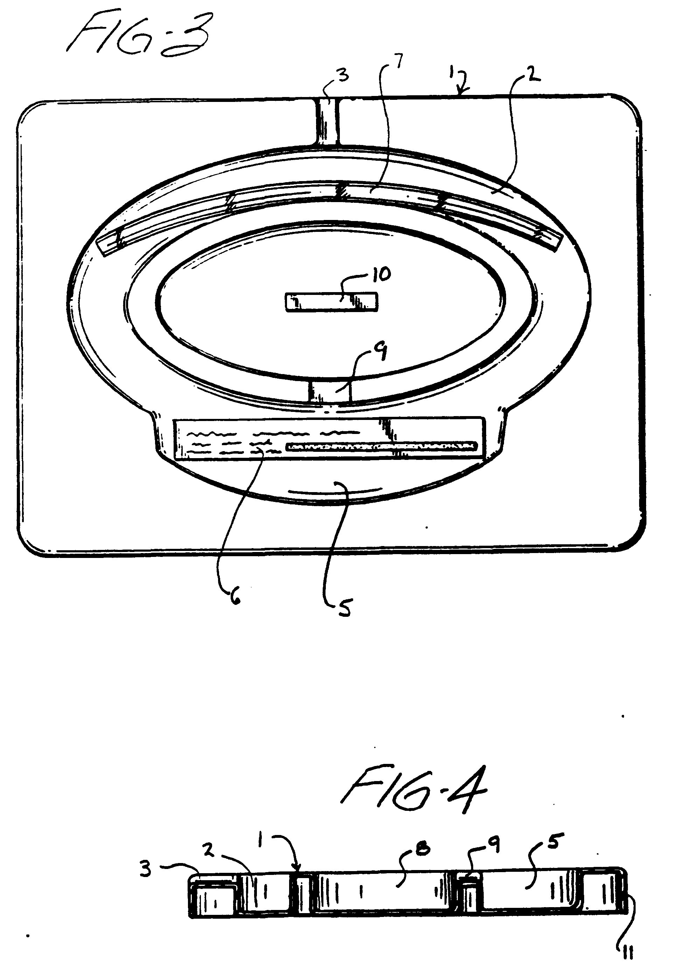

Referring now to FIGS. 1 and 2, a test pack of the present invention is depicted. The pack has a bottom tray 1 made of thermoformable material such as polycarbonate, polyethylene, polypropylene, polystyrene, PVC, acrylic plastics or polyester. In the preferred embodiment, a white polystyrene is used. The base is formed with an oval annular passage 2. Passage 2 communicates with the atmosphere outside through opening 3 defined by tray 1 and peelable top 4. Tray 1 further defines a space 5 which receives a chemical indicator strip 6.

Strip 6 is provided with an indicator suitable for use with the particular environment to be used in the sterilization process, for example, when used in a hydrogen peroxide plasma sterilizer, the strip would be provided with a chemical which changes color when exposed to the sterilization atmosphere.

Within oval passage 2 but opposite chemical indicator strip 6 is an absorber 7. This provides a load to retard the passage of the atmosphere from outside the package. Absorber 7 may be made of any convenient substance which absorbs the atmosphere without interacting with indicators in the pack. For example, in a hydrogen peroxide atmosphere paper, rubber, nylon, polyurethane or PVC may be used. Rubber tubing is preferred when hydrogen peroxide is used either as an active sterilant or a chemical precursor.

Tray 1 defines a blind reservoir 8 which communicates with passage 2 via connecting opening 9. Reservoir 8 contains a biological indicator 10 such as a spore strip or self-contained biological indicator (one with spores and a culture medium in place). A spore strip is preferred. Peelable top 4 is made of a clear plastic film or foil. Although a clear polyester is used in the preferred embodiment, polycarbonate, polyethylene, polypropylene, polystyrene, PVC, acrylic plastics, nylon or an opaque aluminium foil may be used. The top 4 is held in place by a suitable adhesive. The adhesive preferably seals the top 4 to tray 1. One corner may be left without adhesive near the corner portion to permit ease of grasping top 4 to separate it from tray 1 for access to the indicators.

The tray 1 has a depending skirt 11 to prevent curling of the corners. The tray is thermoformed and receives absorber 7, chemical indicator strip 6 and biological indicator 10. The peelable top is then sealed to the upper surface of tray 1 so the only communication between passage 2 and the outside is through opening 3.

In use, the indicator is placed within the chamber of a sterilizer along with the objects to be sterilized.

During the operation of the sterilizer, a portion of the atmosphere enters passage 2 through opening 3. Absorber 7 retards the active species of the atmosphere from traveling around to the chemical indicator strip 6. As the process continues, the atmosphere permeates past absorber 7 and around to strip, 6 causing the strip to indicate it has been in contact with the atmosphere. As the sterilization process continues, a portion of the sterilization atmosphere permeates through connecting opening 9 and into the blind reservoir 8 where it acts on the biological indicator.

If sufficient sterilization atmosphere reached the biological indicator to kill all the spores present, the sterilization is deemed adequate. To determine the kill rate, the indicator is removed and incubated (with an appropriate nutrient media) to determine spore survival. To remove the indicator, the top 4 is peeled away from tray 1 to access the indicator.

The test pack of the present invention provides an initial indication of completed sterilization cycle via the chemical indicator. The biological indicator is then used to determine whether sufficient sterilization atmosphere was provided for sufficient time to cause sterilization.

## Claims

1. A test pack for a sterilization procedure wherein a sterilization atmosphere of active species is created and contacted with an object to be sterilized, comprising:
(a) a housing defining an inner volume and an opening (3) for communication between said inner volume and the outside of said housing;
(b) a divider which divides said inner volume into two portions comprising:
(i) a first portion of said inner volume defining a passage (2) between said opening and said second portion of said inner volume; and
(ii) said second portion defining a blind reservoir (8) having a communication port (9) between said reservoir and said first portion; said reservoir and said first portion;
(c) an absorber (7) positioned within said passage (2) to retard passage of said sterilization atmosphere through said passage (2) during the sterilization procedure;
(d) a chemical indicator (6) within said housing and in communication with said passage (2) to indicate contact of the indicator (6) with the sterilization atmosphere; and
(e) a biological indicator (10) positioned within said blind reservoir (8), biological indicator (10) including microorganisms which are capable of being incubated to show whether the sterilization procedure was incomplete.

2. The test pack of Claim 1 wherein said biological indicator (10) is a spore strip.

3. The test pack of Claim 1 or Claim 2 wherein said biological indicator (10) is self-contained with a culture medium for incubation after exposure to the sterilization atmosphere.

4. The test pack of any one of Claims 1 to 3 wherein the absorber (7) is a rubber tube.

5. The test pack of any one of Claims 1 to 4 wherein said first portion is an oval annulus which encircles said second portion.

6. The test pack of any one of Claims 1 to 5 wherein said housing is formed of a thermoformable material.

7. The test pack of Claim 6 wherein said housing formed of a tray (1) having a top (4) peelably sealed to said tray (1) to define said inner volume.

8. The test pack of Claim 7 wherein said top (4) is clear.

9. The test pack of Claim 7 wherein (a) said top (4) is polyester; (b) said tray (1) is polystyrene; (c) said absorber (7) is rubber tubing; and (d) said biological indicator (10) is a spore strip.

## Patentansprüche

1. Testpackung für ein Sterilisationsverfahren, bei der eine Wirkstoff-Sterilisationsatmosphäre gebildet und mit dem zu sterilisierenden Gegenstand in Kontakt gebracht wird, umfassend:
(a) ein Gehäuse, das ein inneres Volumen und eine Öffnung (3) zur Kommunikation zwischen dem inneren Volumen und dem Äußeren des Gehäuses definiert;
(b) einen Raumteiler, der das innere Volumen in zwei Teile teilt, umfassend:
(i) einen ersten Teil des inneren Volumens, der einen Durchtritt (2) zwischen der Öffnung und dem zweiten Teil des inneren Volumens definiert; und
(ii) den zweiten Teil, der ein Sackgassen-Reservoir (8) mit einer Kommunikationsöffnung (9) zwischen dem Reservoir und dem ersten Teil definiert;
(c) ein Absorptionsmittel (7), das innerhalb des Durchtritts (2) angeordnet ist, um den Durchtritt der Sterilisationsatmosphäre durch den Durchtritt (2) während des Sterilisationsverfahrens zu hemmen;
(d) einen chemischen Indikator (6) innerhalb des Gehäuses und in Kommunikation mit dem Durchtritt (2), um einen Kontakt des Indikators (6) mit der Sterilisationsatmosphäre anzuzeigen; und
(e) einen biologischen Indikator (10), der innerhalb des Sackgassen-Reservoirs (8) angeordnet ist, wobei der biologische Indikator (10) Mikroorganismen umfaßt, die inkubierbar sind, um nachzuweisen, ob das Sterilisationsverfahren unvollständig war.

2. Testpackung nach Anspruch 1, wobei der biologische Indikator (10) ein Keimstreifen ist.

3. Testpackung nach Anspruch 1 oder nach Anspruch 2, wobei der biologische Indikator (10) mit einem Kulturmedium zur Inkubation, nachdem er der Sterilisationsatmosphäre ausgesetzt worden ist, ein in sich geschlossenes System bildet.

4. Testpackung nach einem der Ansprüche 1 bis 3, wobei das Absorptionsmittel 7 ein Gummischlauch ist.

5. Testpackung nach einem der Ansprüche 1 bis 4, wobei der erste Teil ein ovaler Ring ist, der den zweiten Teil umgibt.

6. Testpackung nach einem der Ansprüche 1 bis 5, wobei das Gehäuse aus einem thermoformbaren Material gebildet ist.

7. Testpackung nach Anspruch 6, wobei das Gehäuse aus einer Schale (1) gebildet ist, die einen oberen Teil (4) aufweist, der mit der Schale (1) abziehbar versiegelt ist, wodurch das innere Volumen definiert wird.

8. Testpackung nach Anspruch 7, wobei der obere Teil (4) durchsichtig ist.

9. Testpackung nach Anspruch 7, wobei (a) der obere Teil (4) aus Polyester ist; (b) die Schale (1) aus Polystyrol ist; (c) das Absorptionsmittel (7) ein Gummischlauch ist; und (d) der biologische Indikator (10) ein Keimstreifen ist.

## Revendications

1. Un dispositif de test pour une procédure de stérilisation, dans lequel on crée un environnement de stérilisation d'espèces actives que l'on met en contact avec un objet destiné à être stérilisé, comprenant :
(a) un boîtier définissant un volume intérieur et une ouverture (3) permettant de communiquer entre ledit volume intérieur et l'extérieur dudit boîtier ;
(b) un diviseur qui divise ledit volume intérieur en deux parties comprenant :
(i) une première partie dudit volume intérieur définissant un passage (2) entre ladite ouverture et ladite deuxième partie dudit volume intérieur ; et
(ii) ladite deuxième partie définissant un réservoir aveugle (8) présentant une ouverture de raccordement (9) entre ledit réservoir et ladite première partie ;
(c) un absorbeur (7) situé à l'intérieur dudit passage (2) permettant de retarder le passage dudit environnement de stérilisation à travers ledit passage (2) au cours de la procédure de stérilisation ;
(d) un indicateur chimique (6) situé à l'intérieur dudit boîtier et communiquant avec ledit passage (2) pour témoigner de l'entrée en contact de l'indicateur (6) avec l'environnement de stérilisation ; et
(e) un indicateur biologique (10) placé à l'intérieur dudit réservoir aveugle (8), ledit indicateur biologique (10) contenant des micro-organismes qui sont capables d'être incubés pour déterminer dans quelle mesure la procédure de stérilisation est incomplète.

2. Le dispositif de test selon la Revendication 1, dans lequel ledit indicateur biologique (10) est une bande de spores.

3. Le dispositif de test selon la Revendication 1 ou la Revendication 2, dans lequel ledit indicateur biologique (10) est isolé avec un bouillon de culture pour incubation après exposition à l'environnement de stérilisation.

4. Le dispositif de test selon l'une quelconque des Revendications 1 à 3, dans lequel l'absorbeur (7) est un tube de caoutchouc.

5. Le dispositif de test selon l'une quelconque des Revendications 1 à 4, dans lequel ladite première partie est un anneau oval qui encercle ladite deuxième partie.

6. Le dispositif de test selon l'une quelconque des Revendications 1 à 5, dans lequel ledit boîtier est formé à partir d'un matériau thermoformable.

7. Le dispositif de test selon la Revendication 6, dans lequel ledit boîtier est formé d'un plateau (1) équipé d'un couvercle (4), pelable et hermétiquement relié audit plateau (1) pour définir ledit volume intérieur.

8. Le dispositif de test selon la Revendication 7, dans lequel ledit couvercle (4) est transparent.

9. Le dispositif de test selon la Revendication 7, dans lequel (a) ledit couvercle (4) est du polyester ; (b) ledit plateau (1) est du polystyrène ; (c) ledit absorbeur (7) est un tube de caoutchouc ; et (d) ledit indicateur biologique (10) est une bande de spores.
